# EUROPEAN PATENT SPECIFICATION

(11) **EP 2 210 888 B1**
(45) Date of publication and mention of the grant of the patent: **24.04.2013**
(21) Application number: 10156812.9
(22) Date of filing: 10.05.2006
(51) Int. Cl.: C07D 403/10, A61K 31/4184, A61P 9/10

(54) **Stable micronized candesartan cilexetil and methods for preparing thereof**
Stabiles mikronisiertes Candesartan-Cilexetil und Herstellungsverfahren
Candesartan cilexetil micronisé stable et son procédé de préparation

(30) Priority: 10.05.2005 US 679952 P; 11.05.2005 US 680115 P; 24.05.2005 US 684455 P; 10.08.2005 US 707417 P; 19.08.2005 US 709954 P; 13.09.2005 US 716995 P; 29.09.2005 US 722388 P
(43) Date of publication of application: 28.07.2010
(62) Divisional of application: 06759583.5
(73) Proprietor: TEVA PHARMACEUTICAL INDUSTRIES, LTD., 49131 Petah Tiqva (IL)
(72) Inventor: Kurgan, Ziv, Beer-sheva (IL); Pesachovich, Michael, Givat-shmuel (IL)
(74) Representative: Gallagher, Kirk James

(56) References cited:
- EP-A2- 1 763 525
- WO-A-2005/111021
- WO-A1-00/67892
- US-A- 5 196 444
- MATSUNAGA ET AL.: "Solid-state characterization of candesartan cilexetil (TCV-116): crystal structure and molecular mobility" CHEM. PHARM. BULL., vol. 47, no. 2, February 1999 (1999-02), pages 182-186, XP002957606 ISSN: 0009-2363
- Rote Liste Service GmbH (editor): "Rote Liste 2004" 2004, Editio Cantor Verlag , Aulendorf , XP002594024 * Entries 17154 (Atacand Tabletten) and 17155 (Blopross Tabletten). *
- BLACK ET AL.: "Laser-based techniques for particle-size measurement: a review of sizing methods and their industrial applications" PROGRESS IN ENERGY AND COMBUSTION SCIENCE, vol. 22, no. 3, 1996, pages 267-306, XP004068954 ISSN: 0360-1285
- Malvern Instruments: "MAN 0101 Issue 1.3 - Getting Started Manual for Mastersizer X and Mastersizer S." August 1997 (1997-08), Malvern Instruments Ltd. , Malvern (UK) , XP002594025 * the whole document *
- Malvern Instruments: "Mastersizer S, User Manual MAN 0096 Issue 1.0", November 1994, Malvern Instruments Ltd., Malvern (UK), chapter 2, pages 11-13, chapter 8, pages 87-90.
- Zeng: "Particulate Analysis - Particle Size"; "Chapter 11" In: Storey, Ymen (eds.): "Solid State Characterization of Pharmaceuticals", 31 May 2011 (2011-05-31), John Wiley & Sons, Chichester ISBN: 9781405134941 pages 388-425,
- Rawle: Technical Paper - Basic Principles of Particle Size Analysis, Malvern Intruments, pages 1-8.
- US Pharmacopoeia, vol 23 (1995), chapter 941: x-ray diffraction, pages 1843-1844.
- Connolly: Introduction to X-ray Powder Diffraction, 2007, pages 1-9. Accessible from the internet under: http://epswww.unm.edu/xrd/xrdclass/01-XRD-I ntro.pdf

## Description

### FIELD OF INVENTION

The present invention encompasses stable candesartan cilexetil of fine particle size, processes for its preparation and pharmaceutical compositions thereof.

### BACKGROUND OF THE INVENTION

Candesartan (CNS) is a potent, long-acting, selective AT₁ subtype angiotensin II receptor antagonist. Candesartan is a useful therapeutic agent for treating circulatory system diseases such as hypertensive diseases, heart diseases (e.g. hypercardia, heart failure, cardiac infarction, etc.), strokes, cerebral apoplexy, and nephritis, among others. Candesartan meets the requirement of high potency but it is poorly absorbed when administered orally. Therefore, the prodrug candesartan cilexetil was developed. During absorption from the gastrointestinal tract candesartan cilexetil is rapidly and completely hydrolyzed to candesartan. The chemical name for candesartan is: 2-ethoxy-1-[[2'-(1H-tetrazol-5-yl)biphenyl-4-yl]methyl]-1H-benzimidazole-7-carboxylic acid. The chemical name for candesartan cilexetil is (±)-1-[[(cyclohexyloxy)carbonyl]oxy]ethyl-2-ethoxy-1-[[2'-(1H-tetrazol-5-yl)[1,1'biphenyl]-4-yl]methyl]-1H-benzimidazole-7-carboxylate.

Candesartan cilexetil is a white to off-white powder and is sparingly soluble in water and in methanol. Although candesartan cilexetil contains an asymmetric center in the ester portion of the molecule, it is sold as the racemic mixture.

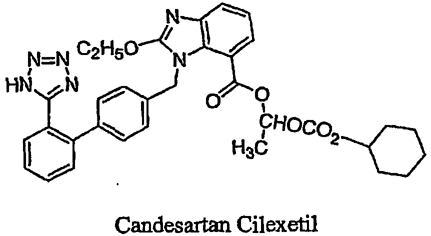

Angiotensin II is formed from angiotensin I in a reaction catalyzed by angiotensin-converting enzyme (ACE, kininase II). Angiotensin II is the principal pressor agent of the renin-angiotensin system, with effects that include vasoconstriction, stimulation of synthesis and release of aldosterone, cardiac stimulation, and renal reabsorption of sodium. Angiotensin II help maintain constant blood pressure despite fluctuations in a person's state of hydration, sodium intake and other physiological variables. Angiotensin II also performs the regulatory tasks of inhibiting excretion of sodium by the kidneys, inhibiting norephedrine reuptake and stimulating aldosterone biosynthesis. Candesartan blocks the vasoconstrictor and aldosterone secreting effects of angiotensin II by selectively blocking the binding of angiotensin II to the AT₁ receptor in many tissues, such as vascular smooth muscle and the adrenal gland. By inhibiting angiotensin II binding to AT₁ receptors, candesartan disrupts the vasoconstriction mediated by AT₁ receptors. Blocking vasoconstriction by angiotensin II has been found to be beneficial to patients with hypertension. The United States Food and Drug Administration has approved candesartan for the treatment of hypertension alone or in combination with other antihypertensive agents.

U.S. Patent No. 5,196,444 (the '444 patent) relates to one crystalline form of candesartan cilexetil, the C-type crystal, which has the following lattice spacings: 3.5, 3.7, 3.8, 4.0, 4.1, 4.3, 4.4, 4.6, 4.8, 5.1, 5.2, 6.9, 7.6, 8.8, 9.0 and 15.9 Å, with varying peak intensities. The C-type crystals claimed by US'444, are said to be stable by heating, but no data is provided in support.

This crystalline form is also mentioned in Chem. Pharm. Bull., 47 (2), 182-186 (1999), where it is referred to as Form I, and exhibits the same x-ray characterization as the C-type crystals of the '444 patent

The commercial tablet: Atacand® appears to contain the active ingredient in reduced particle sizes. Small particles size is usually achieved by milling or micronization. Particle size reduction of Candesartan cilexetil, however, showed to have an adverse effect on its chemical stability.

In view of the foregoing, there is a need to produce a stable Candesartan cilexetil of fine particle size.

### SUMMARY OF THE INVENTION

In one embodiment, the present invention encompasses stable candesartan cilexetil of fine particle size as defined in claim 1. It has been surprisingly observed that desethyl-candesartan (desethyl-CNS) within the stable candesartan cilexetil does not increase to more than about 0.1%w/w by HPLC that relative to the initial amount of candesartan cilexetil, when the stable candesartan cilexetil is maintained at a temperature of about 55°C for at least 2 weeks.

The candesartan cilexetil is the crystalline Form I, characterized by x-ray diffractogram having peaks at about 5.6, 9.8, 17.0, 18.5, and 22.2 ±0.2 degrees two-theta. This stable candesartan cilexetil of fine particle size may be further characterized by a DSC thermogram having an endotherm with a peak temperature of at least about 158.0°C followed by an exothermal peak caused by decomposition.

Another embodiment of the invention encompasses a process for the preparation of stable micronized candesartan cilexetil of fine particle size as defined in claim 1, comprising slurrying a sample ofcandesartan cilexetil of fine particle size in a suitable solvent for a suitable amount of time, and recovering stable candesartan cilexetil of fine particle size, wherein desethyl-candesartan (desethyl-CNS) within the stable candesartan cilexetil does not increase to more than about 0.1%w/w by HPLC relative to the initial amount of candesartan cilexetil, when the stable candesartan cilexetil is maintained at a temperature of about 55°C for at least 2 weeks.

The solvent is a C₁-C₄ alcohol, and more preferably methanol or ethanol. The slurrying is performed at a temperature of at least about 15°C. This process' may be also used in order to further stabilize candesartan cilexetil.

The invention also encompasses pharmaceutical compositions comprising stable candesartan cilexetil of fine particle size as defined in claim 1 and a pharmaceutically acceptable excipient, and methods of treating circulatory system diseases using the same. The candesartan cilexetil is Form I.

### BRIEF DESCRIPTION OF THE FIGURES

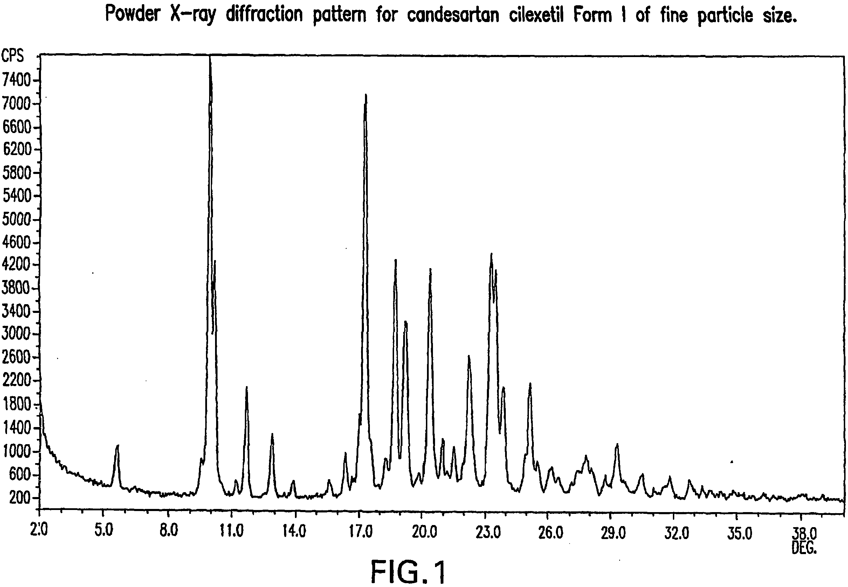

Figure 1 illustrates the powder X-ray diffraction pattern for candesartan cilexetil Form I of fine particle size.
Figure 2 illustrates the powder X-ray diffraction pattern for candesartan cilexetil Form I of fine particle size after slurry in the suitable solvent.

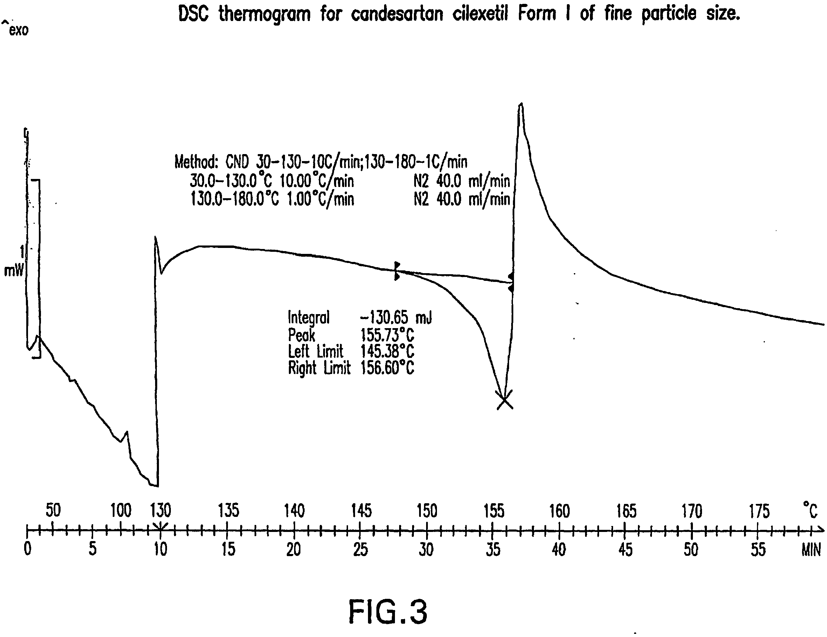

Figure 3 illustrates the DSC thermogram for candesartan cilexetil Form I of fine particle size.
Figure 4 illustrates the DSC thermogram for candesartan cilexetil Form I of fine particle size obtained after slurry.

### DETAILED DESCRIPTION OF THE INVENTION

Particles size can affect the solubility properties of a compound, like candesartan cilexetil. Particle size reduction is one method of formulating materials having low solubility, like candesartan cilexetil. Particle size reduction can also increase a compound's dissolution rate, and hence, its bioavailability. Not to be limited by theory, the enhanced dissolution rate is achieved with the increase in the surface area as a result of particle size reduction. Sometimes the rate of dissolution of a poorly soluble drug is the rate limiting factor in its rate of absorption by the body. However, these drugs may be more readily bioavailable if administered in a finely divided state.

Particle size also can affect how freely crystals or a powdered form of a drug will flow past each other which has consequences in the production process of pharmaceutical products containing the drug.

As used herein, the term "fine particle size" refers to a sample comprising particles having a maximum diameter of no more than about 20 µm.

As used herein, the term "stable" in reference to candesartan cilexetil means candesartan cilexetil wherein the level of the desethyl-candesartan (desethyl-CNS) impurity does not increase to more than about 0.1 %w/w by HPLC, relative to the candesartan cilexetil, when maintained at a temperature of about 55°C for at least 2 weeks.

The present invention provides stable candesartan cilexetil of fine particle size as defined in claim 1. The candesartan cilexetil is crystalline Form I.

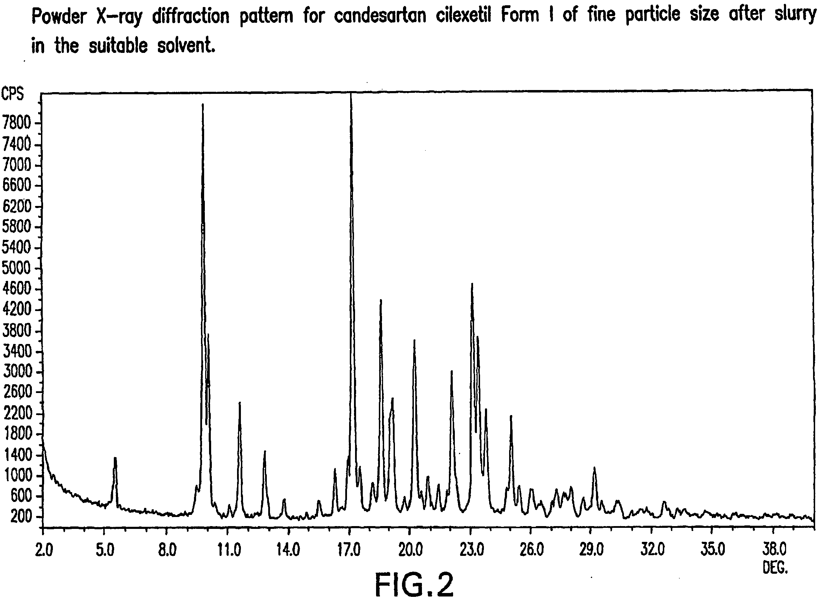

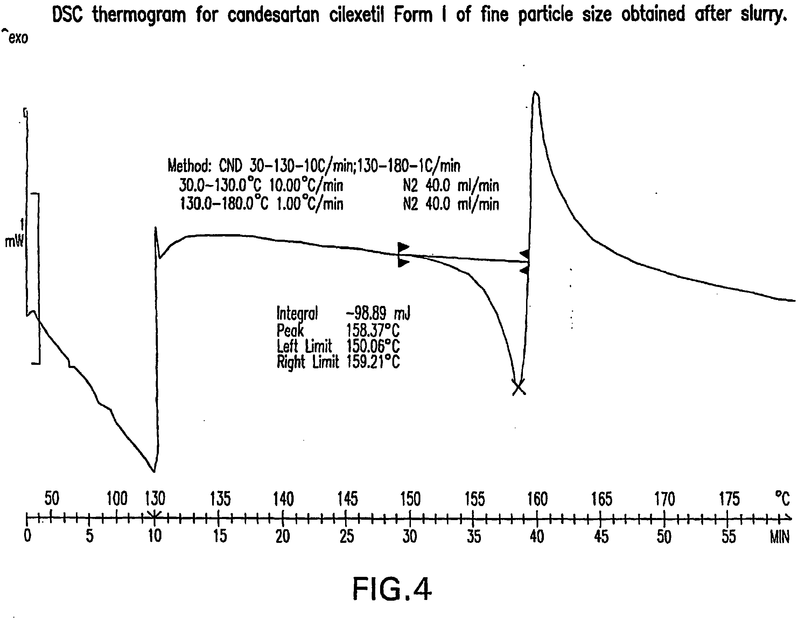

Candesartan cilexetil Form I may be characterized by main x-ray diffraction peaks at about 5.6, 9.8, 17.0, 18.5 and 22.2 ± 0.2 degrees two-theta. Form I is substantially depicted in Figure 2. This stable candesartan cilexetil of fine particle size is further characterized by a DSC thermogram having an endotherm with a peak temperature of at least about 158.0°C followed by an exothermal peak caused by decomposition, substantially as depicted in Figure 4.

The present invention also encompasses processes for the preparation of stable candesartan cilexetil of fine particle size as defined in claim 1. The processes comprise:
a) providing a sample of candesartan cilexetil of fine particle size;
b) slurrying the sample in at least one C₁-C₄ alcohol for about 16 to about 48 hours;
c) recovering stable candesartan cilexetil of fine particle size.

Preferably, the C₁-C₄ alcohol is methanol or ethanol. Preferably, the slurrying in step b) is performed at a temperature of at least about 15°C, more preferably, at a temperature of between about 15°C to about 50°C, and most preferably, at a temperature of between about 25°C to about 35°C. Preferably, the sample is slurried for about 20 to about 30 hours.

The stable candesartan cilexetil of fine particle size as defined in claim 1 may be recovered by any method known in the art, such as cooling the sample, filtering out the solvent, washing the particles, preferably with the solvent added in step a), and drying the particles, preferably at a temperature of about 60°C under reduced pressure.

The candesartan cilexetil of fine particle size provided in step a) may be obtained directly from the synthesis of candesartan cilexetil, or by comminuting a candesartan cilexetil sample in order to decrease their size to a maximum diameter of no more than about 20µm.

Comminution of the candesartan cilexetil of fine particle size may be performed by any known methods of particle size reduction starting with crystals, powder aggregates and course powder of either crystalline or amorphous candesartan cilexetil. The principal operations of conventional size reduction are milling of a feedstock material and sorting of the milled material by size.

A fluid energy mill, or micronizer, is an especially preferred type of mill for its ability to produce particles of small size in a narrow size distribution. The feedstock should be provided in an average particle size range of about 12 to 20 µm which may be achieved using a conventional ball, roller or hammer mill if necessary. As those skilled in the art are aware, fluid energy mills use the kinetic energy of collision between particles suspended in a rapidly moving fluid (typically air) stream to cleave the particles. The suspended particles are injected under pressure into a recirculating gas stream. Smaller particles are carried aloft inside the mill and swept into a vent and are collected. The vent may be connected to a particle size classifier such as a cyclone. Fluid energy mills are designed so that particles are classified by mass. Only particles with a momentum in a certain range will enter the vent and be collected. Centrifugal forces serve to classify the particles in a fluid energy mill. When milled in another type of mill, a powder composition according to this invention can be produced using cyclonic or centrifugation separation techniques.

Candesartan cilexetil samples of fine particle size were slurried as described above. The peak temperature and the stability of the slurried sample were studied for two weeks. The results are summarized in Table 1. The values are reported as weight percent (w/w%) as determined by HPLC.

**Table 1: Correlation between stability results of samples of Candesartan cilexetil and their peak temperature (measured by DSC)**

| Preparation | Peak temperature [°C] | Time | CNS desethyl | 1-N-ethyl CNS | 2-N-ethyl CNS |
|---|---|---|---|---|---|
| Fine particle size | 153.9 | T=0 | 0.24 | 0.04 | 0.17 |
| | | 2 weeks | 0.41 | 0.08 | 0.29 |
| Slurried fine particle size sample | 158.9 | T=0 | 0.08 | 0.02 | 0.06 |
| | | 2 weeks | 0.10 | 0.02 | 0.07 |
| Fine particle size | 155.7 | T=0 | 0.04 | LTDL | LTDL |
| | | 2 weeks | 0.14 | LTDL | 0.09 |
| Slurried fine particle size sample | 158.4 | T=0 | 0.03 | LTDL | LTDL |
| | | 2 weeks | 0.06 | LTDL | LTDL |

The results in Table 1 demonstrate that the peak temperature and the stability of the fine particle size samples increase significantly after the slurrying.

The duration of the slurrying is also an important factor for the stability of candesartan cilexetil, as shown in Table 2. The values are reported as weight percent (w/w%) as determined by HPLC.

**Table 2a: Correlation between time of slurry and peak temperature of Candesartan cilexetil, measured by DSC and stability**

| Time of slurry [hr] | Peak temperature [°C] | Time | CNS-desethyl |
|---|---|---|---|
| 2 | 157.3 | T=0 | 0.03 |
| | | 2 weeks | 0.07 |
| 20 | 160.2 | T=0 | LTDL |
| | | 2 weeks | 0.03 |

**Table 2b: Correlation between time of slurry and peak temperature of Candesartan cilexetil of fine particle size, measured by DSC and stability**

| Time of slurry [hr] | Peak temperature [°C] | Time | CNS-desethyl |
|---|---|---|---|
| 3 | 159.6 | T=0 | 0.07 |
| | | 2 weeks | 0.10 |
| 6 | 160.2 | T=0 | 0.06 |
| | | 2 weeks | 0.07 |
| 9 | 160.5 | T=0 | 0.05 |
| | | 2 weeks | 0.06 |
| 13 | 161.2 | T=0 | 0.05 |
| | | 2 weeks | 0.06 |
| 23 | 161.3 | T=0 | 0.04 |
| | | 2 weeks | 0.05 |

The results in Tables 2 (a) and (b) demonstrate that by increasing the time of slurrying, the stability of the sample, measured by peak temperature and CNS-desethyl level, is increased.

The size of candesartan cilexetil particles of the present invention may be determined by any method known in the art, such as laser diffraction, sieve analysis, microscope observation, sedimentation etc.

The present invention further provides a pharmaceutical composition comprising stable candesartan cilexetil of fine particle size and a pharmaceutically acceptable excipient. Also provided are methods of treating circulatory system diseases using the same. The stable fine particle size candesartan cilexetil in the pharmaceutical composition yields a stable pharmaceutical composition. Preferably, the candesartan cilexetil is crystalline Form 1.

The pharmaceutical composition comprising the stable fine particle size candesartan cilexetil of the invention may be prepared by using a diluent or an excipient such as carriers, fillers, bulking agents, binders, wetting agents, disintegrating agents, surface active agents, lubricants, and the like. The pharmaceutical composition can include at least one diluent or excipient. For the pharmaceutical compositions, various types of administration unit forms can be selected depending on the therapeutic purpose, for example tablets, pills, powders, liquids, suspensions, emulsions, granules, capsules, suppositories, injection preparations (solutions and suspensions), and the like. Any excipient commonly known and used widely in the art can be used in the pharmaceutical composition. Carriers include, but are hot limited to, lactose, white sugar, sodium chloride, glucose, urea, starch, calcium carbonate, kaolin, crystalline cellulose, and silicic acid. Binders include, but are not limited to, water, ethanol, propanol, simple syrup, glucose solutions, starch solutions, gelatin solutions, carboxymethyl cellulose, shelac, methyl cellulose, potassium phosphate, and polyvinylpyrrolidone. Disintegrating agents include, but are not limited to, dried starch, sodium alginate, agar powder, laminalia powder, sodium hydrogen carbonate, calcium carbonate, fatty acid esters of polyoxyethylene sorbitan, sodium laurylsulfate, monoglyceride of stearic acid, starch, and lactose. Disintegration inhibitors include, but are not limited to, white sugar, stearin, coconut butter, and hydrogenated oils. Absorption accelerators include, but are not limited to, quaternary ammonium base, and sodium laurylsulfate. Wetting agents include, but are not limited to, glycerin, and starch. Adsorbing agents include, but are not limited to, starch, lactose, kaolin, bentonite, and colloidal silicic acid. Lubricants used include, but are not limited to, purified talc, stearates, boric acid powder, and polyethylene glycol. Tablets can be further coated with commonly known coating materials such as sugar coated tablets, gelatin film coated tablets, tablets coated with enteric coatings, tablets coated with films, double layered tablets, and multi-layered tablets.

When tableting the pharmaceutical composition, any commonly known excipient used in the art can be used. For example, carriers include, but are not limited to, lactose, starch, coconut butter, hardened vegetable oils, kaolin, and talc. Binders include, but are not limited to, gum arabic powder, tragacanth gum powder, gelatin, and ethanol. Disintegrating agents include, but are not limited to, agar, and laminalia.

For the purpose of shaping the pharmaceutical composition in the form of suppositories, any commonly known excipient used in the art can be used. For example, excipients include, but are not limited to, polyethylene glycols, coconut butter, higher alcohols, esters of higher alcohols, gelatin, and semisynthesized glycerides.

When preparing injectable pharmaceutical compositions, solutions and suspensions are sterilized and are preferably made isotonic to blood. Injection preparations may use carriers commonly known in the art. For example, carriers for injectable preparations include, but are not limited to, water, ethyl alcohol, propylene glycol, ethoxylated isostearyl alcohol, polyoxylated isostearyl alcohol, and fatty acid esters of polyoxyethylene sorbitan. One of ordinary skill in the art can easily determine with little or no experimentation the amount of sodium chloride, glucose, or glycerin necessary to make the injectable preparation isotonic. Additional ingredients, such as dissolving agents, buffer agents, and analgesic agents may be added. If necessary, coloring agents, preservatives, perfumes, seasoning agents, sweetening agents, and other medicines may also be added to the desired preparations during the treatment of circulatory system diseases.

Methods of administration of a pharmaceutical composition for treating circulatory system diseases of the present invention are not specifically restricted, and can be administered in various preparations depending on the age, sex, and symptoms of the patient. For example, tablets, pills, solutions, suspensions, emulsions, granules and capsules may be orally administered. Injection preparations may be administered individually or mixed with injection transfusions such as glucose solutions and amino acid solutions intravenously. If necessary, the injection preparations are administered singly intramuscularly, intracutaneously, subcutaneously or intraperitoneally. Suppositories may be administered into the rectum.

The amount of candesartan cilexetil contained in a pharmaceutical composition for treating circulatory system diseases according to the present invention is not specifically restricted, however, the dose should be sufficient to treat, ameliorate, or reduce the symptoms associated with the circulatory system disease. The dosage of a pharmaceutical composition for treating circulatory system diseases according to the present invention will depend on the method of use, the age, sex, and condition of the patient. Typically, about 4 mg to 32 mg of candesartan cilexetil may be contained in an administration form unit.

Having described the invention with reference to certain preferred embodiments, other embodiments will become apparent to one skilled in the art from consideration of the specification. The invention is further defined by reference to the following examples describing in detail the crystals and processes for making the crystals of the invention. It will be apparent to those skilled in the art that many modifications, both to materials and methods, may be practiced without departing from the scope of the invention.

### EXAMPLES

Candesartan cilexetil Form I crystals of fine particle size were identified using Scintag X-ray powder diffractometer model X'TRA, Cu-tube solid state detector. The sample holder was a round standard aluminum sample holder with rough zero background quartz plate with a cavity of 25 (diameter)*0.5 mm (depth). The scanning parameters were range: 2-40 and in some cases 2-30 degrees two-theta; scan mode: continuous scan; step size: 0.05 deg.; and a rate of 3 deg/min.

DSC analysis was done on a Mettler 821 Star e. The weight of the samples was about 5 mg; the samples were scanned at a rate of 10°C/min from 30°C to 130°C and at a rate of 1°C/min from 130°C to 180°C. The oven was constantly purged with nitrogen gas at a flow rate of 40 ml/min. Standard 40 µl aluminum crucibles covered by lids with 3 holes were used.

Particle size measurements were done on a Malvern Mastersizer S instrument. The solvent silicon fluid Silicaid F-10 was used for measurements. The sample amount was about 0.1 g. The suspension was prepared by vortex for 10 seconds and by sonication for 2 minutes. The measurements were done after 15 seconds recirculation at speed rate 2500 rpm.

### HPLC method:

| | | | |
|---|---|---|---|
| Column & Packing: | RP18 150*4.6mm, 5µ | | |
| Buffer: | 0.10% (v/v) Triethylamine in water adjusted to pH 3.5 with 85% H₃PO₄ | | |
| Eluent A: | Buffer | | |
| Eluent B: | Acetonitrile | | |

| Gradient | Time | % Eluent A | % Eluent B |
|---|---|---|---|
| | Initial time | 30% | 70% |
| | 10 min | 30% | 70% |
| | 15 min | 10% | 90% |
| | 30 min | 5% | 95% |
| Equilibrium time: | 10 min | | |
| Sample volume: | 10 µL | | |
| Flow Rate: | 1.0 mL/min | | |
| Detector: | 215 nm | | |
| Diluent | Acetonitrile | | |

The detection limit was 0.02%. An example sample solution preparation was carried out by accurately preparing a 0.8 mg/mL solution of candesartan.

### Example 1: Preparation of Candesartan Cilexetil

A solution of Trityl Candesartan Cilexetil (TCS, 1000 g,1172 mmol), Toluene (3000 mL), Methanol (6000 mL) and Water (50 mL) was refluxed for about 3-4 h (HPLC control), the solvents were evaporated at 50°C under reduce pressure to give a residue as a viscous oil. The residue was dissolved at 50°C in a mixture of Toluene/Methanol (2960 g, 95:5, w/w). The mixture was then cooled to (-5)°C to (5)°C and kept at this temperature for about 12 h. The precipitated solids were filtered off, washed on the filter with cold Toluene (1000 mL) and then dried at 60°C under reduced pressure to give crude candesartan cilexetil Form I (∼600 g L.O.D = 17%).

### Example 2: Preparation of Candesartan Cilexetil Form I

601 g of CNS-Crude with LOD<15% were slurried at 20-30 °C in absolute ethanol (3174 mL 6V) for 20-30 hours. The precipitated solids were filtered off, washed with cold Absolute ethanol (550 mL) to give 644 g wet material (LOD=30-40% ∼80 %), which then 429 g were dried at 60°C under reduced pressure to give candesartan cilexetil Form I (∼274.5 g L.O.D.=0.12%).

### Example 3: Preparation of Stable Candesartan Cilexetil Form I of fine particle size

75 g of micronized CNS-Cryst were slurried at 25°C in Ethanol Absolute (500mL 6V) for 24 hours. The precipitated solids were filtered off, and then were dried at 60°C under reduce pressure to give stable candesartan cilexetil Form I. Desethyl-CNS: 0.08%.

The stability of the starting material was tested by maintaining a sample containing micronized CNS-Cryst in an oven at 55°C for 2 weeks, after which the level of desethyl-CNS increased from 0.24% to 0.41% w/w by HPLC.

Similarly, the stability of the obtained product was tested, and the level of desethyl-CNS increased to 0.10% w/w by HPLC.

### Example 4: Preparation of Stable Candesartan Cilexetil Form I of fine particle size

5.8 Kg of CNS-Crude dry (LOD<15%) were slurried at 15-25°C in absolute ethanol (30L 6V) for 2-3 hours. The slurry was then cooled to (-5) - (5)°C, and kept at this temperature for 2-3 hr, the precipitated solids were filtered off, washed with cold absolute ethanol (5L) and then dried at 50°C under reduced pressure to give candesartan cilexetil Form I (∼4.15 Kg L.O.D.=0.76%). Desethyl-CNS: 0.03% by area HPLC. Maintaining the dried product for 2 weeks increased the desethyl-CNS level to 0.07% w/w by HPLC.

1.75 Kg of the obtained material was micronized to give 1.55 Kg of micronized material. Desethyl-CNS: 0.04% w/w by HPLC. Maintaining the micronized product for 2 weeks increased the desethyl-CNS level to 0.14% w/w by HPLC.

1.36 Kg of the micronized material were slurried at 25°C in absolute ethanol (8.1L 6V) for 44 hours. The precipitated solids were filtered off and dried at 60°C under reduced pressure to give stable micronized candesartan Form I (∼1.24 Kg L.O.D.=0.10%). Desethyl-CNS: 0.03% by w/w HPLC. Maintaining the micronized product for 2 weeks increased the desethyl-CNS level to 0.06% area by HPLC.

### Example 5:

Micronized candesartan (70 g, Sample 1) was slurried at 50°C in absolute ethanol (420 mL, 6 vol.) for 31 hours. Thereafter, the precipitated solids were collected by filtration and dried at 60°C under reduce pressure to yield micronized candesartan (Product) having desethyl-CNS in 0.05% w/w by HPLC.

The starting micronized candesartan and the product candesartan were tested for stability in an oven at 55°C for 2 weeks. Table 3 below summarizes the results. The values are reported as weight percent (w/w%) as determined by HPLC.

| Table 3. Micronized Candesartan Stability Test. | | | | |
|---|---|---|---|---|
| Sample | Description | CNS desethyl | 1-N-ethyl CNS | 2-N-ethyl CNS |
| Sample 1 | Micronized starting material | 0.04 | LTDL | LTDL |
| Sample 1B | Sample 1 after 2 weeks at 55°C | 0.20 | 0.04 | 0.15 |
| Product | After re-slurry at 50°C for 31 hrs | 0.05 | LTDL | 0.03 |
| Product B | Product after 2 weeks at 55°C | 0.05 | LTDL | 0.03 |

Table 4 illustrates that the starting micronized candesartan partly decomposed to the 1N-Et and 2N-Et products.

### Comparative Example 6:

An accelerated stability study shows that the active ingredient of the commercially available tablet Atacand® is not stable. A sample of Atacand® was submitted to a stability study. After 2 weeks at 55°C, the impurity level of the tablet, namely the CNS' desethyl (1-N-ethyl CNS and 2-N-ethyl CNS) significantly increased. The structures of the following impurities are as follows:

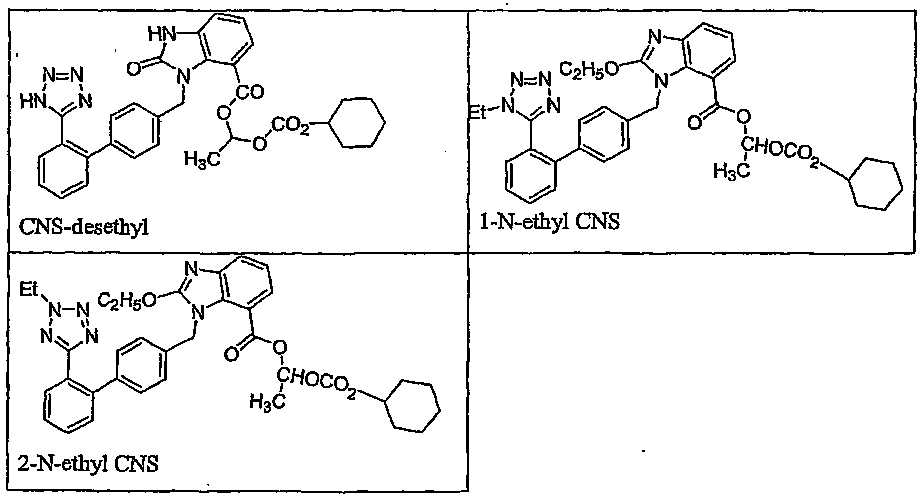

The results are summarized in Table 4, the values in the table are by weight percent (w/w%) as determined by HPLC.

**Table 4: Stability results ofAtacand® 32 mg tablets (Lot No. 5289) at 55°C**

| Time | CNS desethyl | 1-N-ethyl CNS | 2-N-ethyl CNS |
|---|---|---|---|
| T=0 | 0.11 | LTDL | 0.06 |
| 2 weeks | 0.22 | 0.07 | 0.21 |

| | | | |
|---|---|---|---|
| *LTDL means less than detection limit | | | |

## Claims

1. Candesartan cilexetil of fine particle size having a maximum diameter of no more than 20µm as measured by a Malvem Mastersizer S instrument, wherein the candesartan cilexetil is micronised and the level of (+)-1-[[(cyclohexyloxy)carbonyl]oxy])ethyl-1-[[2'-(1H-tetrazol-5-yl)(1,1'-biphenyl)-4-yl]methyl]-1H-benzimidazole-2-one-7-carboxylate (desethyl-candesartan) does not increase to more than 0.1%w/w by HPLC relative to the initial amount of candesartan cilexetil, when said candesartan cilexetil is maintained at a temperature of 55°C for at least 2 weeks, wherein HPLC analysis is carried out using:
| | | | |
|---|---|---|---|
| Column & Packing: | RP18 150*4.6mm, 5µ | | |
| Buffer: | 0.10% (v/v) Triethylamine in water adjusted to pH 3.5 with 85% H₃PO₄ | | |
| Eluent A: | Buffer | | |
| Eluent B: | Acetonitrile | | |
| Gradient | Time | % Eluent A | % Eluent B |
|---|---|---|---|
| | Initial Time | 30% | 70% |
| | 10 min | 30% | 70% |
| | 15 min | 10% | 90% |
| | 30 min | 5% | 95% |
| Equilibrium time: | 10 min | | |
| Sample volume: | 10 µL | | |
| Flow Rate: | 1.0 mL/min | | |
| Detector: | 215 nm | | |
| Diluent | Acetonitrile | | |
wherein the candesartan cilexetil is **characterized by** x-ray diffraction peaks at 5.6, 9.8, 17.0, 18.5 and 22.2 ± 0.2 degrees two-theta, as measured using a Scintag X-ray powder diffractometer model X'TRA, Cu-tube solid state detector.

2. The candesartan cilexetil of claim 1, **characterized by** a DSC thermogram having an endotherm with a peak temperature of at least 158.0°Cwherein DSC analysis is done on a Mettler 821 Star e, sample weight of 5 mg in 40 µl aluminium crucibles covered by lids with 3 holes, scanning rate of 10°C/min from 30°C to 130°C and at a rate of 1°C/min from 130°C to 180°C with constant purging with nitrogen gas at a flow rate of 40 ml/min.

3. A process for the preparation of the candesartan cilexetil as defined in any of claims 1 to 2 comprising:
a) providing micronised candesartan cilexetil of fine particle size having a maximum diameter of no more than 20µm;
b) slurrying the sample in at least one C₁-C₄ alcohol for 16 to 48 hours;
c) recovering the candesartan cilexetil.

4. The process of claim 3, wherein the C₁-C₄ alcohol is methanol or ethanol.

5. The process of any one of claims 3-4, wherein step b) is performed at a temperature of at least 15°C.

6. The process of claim 5, wherein the temperature is between 15°C. and 50°C.

7. The process of claim 6, wherein the temperature is between 25°C and 35°C.

8. The process of any of claims 3 to 7, wherein step b) is performed for 20 to 30 hours.

9. A pharmaceutical composition comprising candesartan cilexetil as defined in any of claims 1 to 2 and a pharmaceutically acceptable excipient.

10. Use of candesartan cilexetil as defined in any of claims 1 to 2 for the manufacture of a medicament for the treatment of circulatory system diseases.

11. Candesartan cilexetil as defined in any one of claims 1 to 2 for use in the treatment of circulatory system diseases.

## Patentansprüche

1. Candesartancilexetil mit feiner Teilchengröße mit einem Maximaldurchmesser von nicht mehr als 20 µm, wie gemessen mit einem Malvern Mastersizer-S-Instrument, wobei das Candesartancilexetil mikronisiert ist und der Gehalt an (±)-1-[[(Cyclohexyloxy)carbonyl]oxy]ethyl-1-[[2'-(1H-tetrazol-5-yl)[1,1'-biphenyl]-4-yl]methyl]-1H-benzimidazol-2-on-7-carboxylat (Desethylcandesartan) auf nicht mehr als 0,1 Gew./Gew.-% nach HPLC bezogen auf die Anfangsmenge an Candesartancilexetil ansteigt, wenn das Candesartancilexetil für wenigstens 2 Wochen bei einer Temperatur von 55 °C gehalten wird, wobei die HPLC-Analyse unter Verwendung von:
| | | | |
|---|---|---|---|
| Säule und Packung: | RP18 150*4,6 mm, 5µ | | |
| Puffer: | 0,10% (v/v) Triethylamin in Wasser, mit 85% H₃PO₄ auf pH 3,5 eingestellt | | |
| Eluens A: | Puffer | | |
| Eluens B: | Acetonitril | | |
| Gradient | Zeit | % Eluens A | % Eluens B |
|---|---|---|---|
| | Startzeit | 30 % | 70 % |
| | 10 min | 30 % | 70 % |
| | 15 min | 10 % | 90 % |
| | 30 min | 5 % | 95 % |
| Äquilibrierungszeit | 10 min | | |
| Probenvolumen: | 10 µl | | |
| Flussrate: | 1,0 µl/min | | |
| Detektor: | 215 nm | | |
| Verdünnungsmittel | Acetonitril | | |
durchgeführt wird, wobei das Candesartancilexetil durch Röntgenbeugungsmaxima bei 5,6, 9,8, 17,0, 18,5 und 22,2 ± 0,2 Grad zwei-Theta, wie gemessen unter Verwendung eines Scintag Röntgenpulverdiffraktometers Modell X'TRA, Cu-Röhre, Feststoffdetektor, gekennzeichnet ist.

2. Candesartancilexetil gemäß Anspruch 1, **gekennzeichnet durch** ein DSC-Thermogramm mit einer Endotherme mit einer Maximum-Temperatur von wenigstens 158 °C, wobei die DSC-Analyse auf einem Mettler 821 Star e, Probengewicht 5 mg in 40 µl Aluminiumtiegeln, bedeckt mit Deckeln mit 3 Löchern, Scanrate 10 °C/min von 30 °C bis 130 °C und mit einer Rate von 1 °C/min von 130 °C bis 180 °C unter gleichbleibendem Spülen mit Stickstoffgas mit einer Flussrate von 40 ml/min durchgeführt wird.

3. Verfahren zum Herstellen des Candesartancilexetils gemäß einem der Ansprüche 1 bis 2, umfassend:
a) Bereitstellen von mikronisiertem Candesartancilexetil mit feiner Teilchengröße mit einem Maximaldurchmesser von nicht mehr als 20 µm;
b) 16 bis 48 Stunden Schlämmen der Probe in wenigstens einem C₁-C₄-Alkohol;
c) Einholen des Candesartancilexetils.

4. Verfahren gemäß Anspruch 3, wobei der C₁-C₄-Alkohol Methanol oder Ethanol ist.

5. Verfahren gemäß einem der Ansprüche 3-4, wobei Schritt b) bei einer Temperatur von wenigstens 15 °C durchgeführt wird.

6. Verfahren gemäß Anspruch 5, wobei die Temperatur zwischen 15 °C und 50 °C beträgt.

7. Verfahren gemäß Anspruch 6, wobei die Temperatur zwischen 25 °C und 35 °C beträgt.

8. Verfahren gemäß einem der Ansprüche 3 bis 7, wobei Schritt b) für 20 bis 30 Stunden durchgeführt wird.

9. Pharmazeutische Zusammensetzung, umfassend Candesartancilexetil gemäß einem der Ansprüche 1 bis 2 und einen pharmazeutisch verträglichen Hilfsstoff.

10. Verwendung von Candesartancilexetil gemäß einem der Ansprüche 1 bis 2 für die Herstellung eines Medikaments für die Behandlung von Kreislaufsystem-Erkrankungen.

11. Candesartancilexetil gemäß einem der Ansprüche 1 bis 2 für die Verwendung bei der Behandlung von Kreislaufsystem-Erkrankungen.

## Revendications

1. Candésartan cilexétil de taille de particule fine ayant un diamètre maximal de pas plus de 20 µm tel que mesuré par un instrument Malvern Mastersizer S, le candésartan cilexétil étant micronisé et le taux de (±)-1-[[(cyclohexyloxy)carbonyl]oxy]éthyl-1-[[2'-(1H-tétrazol-5-yl)[1,1'-biphényl]-4-yl]méthyl]-1H-benzimidazole-2-one-7-carboxylate (déséthyl-candésartan) n'augmentant pas à plus de 0,1 % m/m par CLHP par rapport à la quantité initiale de candésartan cilexétil, lorsque ledit candésartan cilexétil est maintenu à une température de 55 °C pendant au moins 2 semaines, l'analyse CLHP étant conduite en utilisant :
| | | | |
|---|---|---|---|
| Colonne et remplissage : | RP18 150x4,6 mm, 5 µ | | |
| Tampon : | 0,10 % (v/v) triéthylamine dans l'eau ajustée à pH 3,5 avec H₃PO₄ 85 % | | |
| Éluant A : | Tampon | | |
| Éluant B : | Acétonitrile | | |
| Gradient | Temps | % Éluant A | % Éluant B |
|---|---|---|---|
| | Temps initial | 30 % | 70 % |
| | 10 min | 30 % | 70 % |
| | 15 min | 10 % | 90 % |
| | 30 min | 5 % | 95 % |
| Temps d'équilibrage : | 10 min | | |
| Volume d'échantillon : | 10 µl | | |
| Débit : | 1,0 ml/min | | |
| Détecteur : | 215 nm | | |
| Diluant | Acétonitrile | | |
le candésartan cilexétil étant **caractérisé par** des pics de diffraction des rayons X à 5,6, 9,8, 17,0, 18,5 et 22,2 ± 0,2 degrés deux-thêta, tels que mesurés en utilisant un diffractomètre de rayons X sur poudre Scintag modèle X'TRA, détecteur à semi-conducteur à tube Cu.

2. Candésartan cilexétil de la revendication 1, **caractérisé par** un thermogramme ACD ayant un endotherme avec une température de pic d'au moins 158,0 °C, l'analyse ACD étant effectuée sur un Mettler 821 Star e, un poids d'échantillon de 5 mg dans des creusets en aluminium de 40 µl recouverts par des couvercles avec 3 trous, une vitesse de balayage de 10 °C/min de 30 °C à 130 °C et une vitesse de 1 °C/min de 130 °C à 180 °C avec purge constante avec du gaz d'azote à un débit de 40 ml/min.

3. Procédé pour la préparation du candésartan cilexétil tel que défini dans l'une quelconque des revendications 1 à 2 comprenant :
a) la fourniture de candésartan cilexétil micronisé de taille de particule fine ayant un diamètre maximal de pas plus de 20 µm ;
b) la mise en suspension concentrée de l'échantillon dans au moins un alcool en C₁-C₄ pendant 16 à 48 heures ;
c) la récupération du candésartan cilexétil.

4. Procédé de la revendication 3, dans lequel l'alcool en C₁-C₄ est le méthanol ou l'éthanol.

5. Procédé de l'une quelconque des revendications 3 à 4, dans lequel l'étape b) est effectuée à une température d'au moins 15 °C.

6. Procédé de la revendication 5, dans lequel la température est comprise entre 15 °C et 50 °C.

7. Procédé de la revendication 6, dans lequel la température est comprise entre 25 °C et 35 °C.

8. Procédé de l'une quelconque des revendications 3 à 7, dans lequel l'étape b) est conduite pendant 20 à 30 heures.

9. Composition pharmaceutique comprenant du candésartan cilexétil tel que défini dans l'une quelconque des revendications 1 à 2 et un excipient pharmaceutiquement acceptable.

10. Utilisation de candésartan cilexétil tel que défini dans l'une quelconque des revendications 1 à 2 pour la fabrication d'un médicament pour le traitement de maladies du système circulatoire.

11. Candésartan cilexétil tel que défini dans l'une quelconque des revendications 1 à 2 pour utilisation dans le traitement de maladies du système circulatoire.
